Europäisches Patentamt

European Patent Office

Office européen des brevets

(11) Veröffentlichungsnummer : **0 369 185 B1**

(12) **EUROPÄISCHE PATENTSCHRIFT**

(45) Veröffentlichungstag der Patentschrift :
15.07.92 Patentblatt 92/29

(51) Int. Cl.⁵ : **A61L 2/20**

(21) Anmeldenummer : **89119458.1**

(22) Anmeldetag : **20.10.89**

(54) **Verfahren zum Abtöten von Keimen.**

(30) Priorität : **12.11.88 DE 3838448**

(43) Veröffentlichungstag der Anmeldung :
**23.05.90 Patentblatt 90/21**

(45) Bekanntmachung des Hinweises auf die
Patenterteilung :
**15.07.92 Patentblatt 92/29**

(84) Benannte Vertragsstaaten :
**AT BE CH DE ES FR GB GR IT LI LU NL SE**

(56) Entgegenhaltungen :
**EP-A- 0 034 869**
**DE-A- 3 015 813**
**FR-A- 2 296 431**
**US-A- 3 947 568**

(73) Patentinhaber : **Singer, Joachim**
**Ziegeleistrasse 2-6**
**W-6901 Mauer (DE)**
Patentinhaber : **Singer, Jürgen**
**Ziegeleistrasse 2-6**
**W-6901 Mauer (DE)**

(72) Erfinder : **Singer, Joachim**
**Ziegeleistrasse 2-6**
**W-6901 Mauer (DE)**
Erfinder : **Singer, Jürgen**
**Ziegeleistrasse 2-6**
**W-6901 Mauer (DE)**

(74) Vertreter : **Schulze, Ilse**
**Patentanwältin Bergstrasse 49**
**W-6900 Heidelberg (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zum Abtöten von Keimen, Keimprodukten, infektiösen Partikeln und dergleichen pathogenen Erregern auf Festkörpern sowie in Zubereitungen verschiedener Zusammensetzungen und zum Haltbarmachen dieser Zubereitungen, insbesondere von kosmetischen und medizinischen Erzeugnissen.

Gesundheitsschädliche Keime, Keimprodukte, Pilze und dergleichen pathogene Erreger können auf kontaminierten Festkörpern sowie in chemischen Erzeugnissen, beispielsweise Kosmetika und Arzneimitteln wachsen. Die kontaminierten Produkte können dann selbst zu gesundheitsschädigenden Erzeugnissen werden. Kosmetische und medizinische Produkte sind in der Regel gute Nährböden für Bakterien, Pilze und dergleichen, so daß es ein Erfordernis ist, diese Produkte zu schützen. Die Gefahr erhöhter Kontamination ist überall da gegeben, wo reinigende, pflegende oder konditionierende Kosmetika, sowie als Salben, Pasten, Cremes oder Emulsionen vorliegende medizinische Erzeugnisse in Tuben, Spendern, Dosen, Flaschen und ähnlichen Verpackungen aufbewahrt und daraus über längere Zeit in kleinen Portionen entnommen werden.

Es ist daher unbestritten, daß alle diese Erzeugnisse vor toxischen Zersetzungsprodukten geschützt werden müssen, da solche Produkte zum Teil zu erheblichen Gesundheitsschädigungen beim Verbraucher führen können.

Es ist allgemein herrschende Lehre, daß ein solcher Schutz nur durch Zugabe von chemischen Konservierungsmitteln möglich ist. Der Stand der Technik kennt eine Vielzahl chemischer Produkte, die die geforderte Aufgabe erfüllen.

Es ist aber bekannt, daß Konservierungsmittel für den Verbraucher nicht unbedenklich sind, insbesondere da sie Allergien hervorrufen können. Daher sind Vorschriften für den Gebrauch von Konservierungsmitteln erlassen. Die Ausbildung allergischer Reaktionen beim Menschen durch Konservierungsstoffe hat jedoch die unterschiedlichsten Ursachen, so daß auch sorgfältige und gewissenhafte Prüfungen und Zulassungskriterien nicht eine vollkommene Unschädlichkeit eines Konservierungsmittels garantieren können. Hinzu kommt, daß die Vielfalt der Kontaminationsmöglichkeiten durch sehr unterschiedlich wirkende pathogene Erreger und Mikroorganismen auch sehr unterschiedliche Konservierungsmittel erfordern, so daß deren Vielzahl leicht unübersehbar wird.

Schließlich ist zu beachten, daß eine Vielzahl der verwendeten Konservierungsmittel nur das Keimwachstum bereits im Erzeugnis vorhandener Keime, die beispielsweise bei der Produktion und beim Abfüllen eingebracht wurden, verhindern und keine vollständige Abtötung der Keime erreichen.

Aufgabe der Erfindung ist es, ein Verfahren der eingangs genannten Art zu schaffen, mit dem nicht nur eine Hemmung des Keimwachstums, sondern auf einfache und wirtschaftliche Weise unter Vermeidung verbleibender toxischer Wirkungen, Keimfreiheit auf Festkörpern und in kosmetischen und medizinischen Erzeugnissen erreicht wird und diese erhalten bleibt.

Diese Aufgabe wird durch ein Verfahren der eingangs genannten Art dadurch gelöst, daß man die Festkörper oder die Zubereitungen in einen mit einem Ventil versehenen Druckgasbehälter einbringt bzw. in diesen abfüllt und anschließend den Behälterinhalt begast.

Zweckmäßige Weiterbildungen des Verfahrens sind in den Unteransprüchen gekennzeichnet.

Es wurde entgegen der bisherigen Meinung der Fachwelt gefunden, daß bei den genannten Anwendungsbereichen auf chemische Konservierungsmittel verzichtet werden kann. Anstelle der üblichen Töpfe, Tuben, Dosen und dergleichen werden die kosmetischen oder medizinischen Produkte in Druckgasbehältern mit bekannten Treibmitteln abgefüllt und diesem Produkt wird ein Gas oder ein Gasgemisch zugefügt. Es wurde überraschenderweise gefunden, daß Proben, die bereits vor zehn Jahren angesetzt worden waren, nach der Entnahme noch einwandfrei waren und keinerlei Zersetzungserscheinungen aufwiesen. Die Zusammensetzung bestand dabei aus extremen und leicht zum Verderben neigenden Stoffen, nämlich Frischei. Es hat sich gezeigt. daß auch besonders schwer abzutötende Keime erfolgreich bekämpft werden können.

Es wurde aber auch gefunden, daß mit dem erfindungsgemäßen Verfahren Festkörper keimfrei und dabei auf einfache Weise steril gemacht werden können. Diese Erkenntnis ist insbesondere für den medizinischen Sektor von Bedeutung, da sich hier das Problem der Kontamination ärztlicher Instrumente und Geräte stellt. Von diesen wird bei jedem Gebrauch absolute Sterilität, also Freiheit von vermehrungsfähigen Keimen, Keimprodukten und infektiösen Partikeln verlangt. Die bekannten Sterilisationsmethoden zeigen nur für den thermostabilen Bereich eine ausreichende Anwendbarkeit. Dabei wird autoklavierend mit gespanntem Dampf bei 1 bar Überdruck und 134° C oder trockener Hitze bei 180° C gearbeitet. Für den thermolabilen Bereich, also Ethylenoxid-, Formaldehyd- bzw. Strahlensterilisationsbehandlung, sind deutliche Einschränkungen der Anwendbarkeit wegen Materialunverträglichkeiten, aber auch insbesondere wegen der Humantoxizität der verwendeten Gase vorhanden.

Mit dem erfindungsgemäßen Verfahren ist eine einfache und problemlse Sterilisation der Festkörper mög-

EP 0 369 185 B1

lich.

Die Erfindung wird anhand von Versuchen näher erläutert.

Zunächst wurde als Grundversuch mit extremen und leicht verderblichen Produkten gearbeitet.

Der Fettphase 70° bis 80° C wurde, wie üblich, heißes Wasser zugegeben:

| Gew.-% | Stoff |
|---|---|
| 3,0 | Paraffinöl |
| 8,0 | Lanolin anhydricum |
| 3,0 | Emulgierwachs |
| 10,0 | Mandelöl süß |
| 0,2 | Vitamin-A-Palmitat |
| 49,6 | Wasser entmineralisiert, 80°C |

In diese auf 30° bis 40° C abgekühlte Creme wurde eine auf kaltem Wege hergestellte Emulsion aus

| | |
|---|---|
| 10,0 | frisches Vollei |
| 10,0 | Sonnenblumenöl |
| 0,2 | Ascorbinsäure |
| 5,0 | Natrium-Lactat 50%-ig |
| 1,0 | Parfümöl |

Die Abfüllung erfolgte in Aerosol-Glasflaschen im Verhältnis 68 g Wirkstoff zu 7,5 g Flüssiggas als Treibmittel. Das Flüssiggas setzte sich 3 : 1 aus Difluordichlormethan : Butan zusammen.

Anschließend wurde die fertige Abfüllung Wirkstoff/Flüssiggas mit $CO_2$ beaufschlagt, und zwar bis 10 bar - aber auch höher. Dieser Druck baut sich, je nach Wirkstoff-Zusammensetzung stark ab, so daß die gesetzlichen Vorschriften ohne weiteres eingehalten werden können.

Diese Zusammensetzung wurde von einem neutralen Institut auf Mikroorganismen untersucht und dabei wurden folgende Ergebnisse gefunden:

Keimzahlbestimmung:

Bakterien    <10/g
Pilze        <10/g
Hefen        <10/g

Diese Ergebnisse gaben Anlaß zu weiteren intensiven Versuchen. Dabei ging es darum, festzustellen, mit welchen Kombinationen von flüssigen Gasen, nämlich Butan, Dimethylether, Propan/Butan, und $CO_2$, $N_2$, $N_2O$ das System für die unterschiedlichsten Keime eine Wirkung zeigt.

Die Creme, mit der die Versuche durchgeführt wurden, wies folgende Bestandteile auf:

| g Gew.-% | Stoff |
|---|---|
| **Fettphase:** | |
| 2,0 | Paraffinöl |
| 3,8 | Emulgierwachs |
| 1,0 | Lanolin anhydricum |
| 1,0 | Bienenwachs |
| 20,0 | Sonnenblumenöl |
| **Wasserphase:** | |
| 70,2 | entmineralisiertes Wasser |
| 2,0 | Glycerin |
| 100,0 % | |

Wie für Emulsionen üblich wurden die Fettphase und die Wasserphase auf 80° C erhitzt und dann zusammengerührt.

Nach Abkühlen auf etwa 40° C wurde die Emulsion homogenisiert.

Alle weiteren Versuchsansätze gingen von dieser Creme-Zusammensetzung aus. Zur Überprüfung der Fragestellung, inwieweit durch das Verfahren eine Abtötung und nicht nur eine Konservierung der Mikro-Organismen erfolgt, wurden von der Creme jeweils 50 g in 100ml Dosen abgefüllt und diese mit den verschiedenen Keimen kontaminiert. Danach wurden die Dosen luftdicht verschlossen und geschüttelt, um eine gleichmäßige Durchmischung zu erzielen. Danach wurden den Dosen die Gase zugesetzt. Von Flüssiggasen, nämlich Butan, Dimethylether sowie gegebenenfalls Propan/Butan und Mischgas, wurde jeweils eine Menge von 3,5 g verwendet. Die anderen Gase, nämlich $CO_2$, $N_2$, $N_2O$, wurden mit einem Druck von 12 bar beaufschlagt. Dieser Druck baute sich bei $CO_2$ und $N_2O$ nach Schütteln stark ab.

Parallel dazu wurde pures Keim-Material in einer Nährbouillon in 100 ml Dosen abgefüllt und genau wie die Cremes begast. Dabei wurden die einzelnen Keimarten miteinander gemischt.

In den folgenden Tabellen 1 und 2 sind die Ergebnisse der ersten Untersuchung mit "Creme" und mit "Bouillon" bezeichnet worden. - Es wurde mit drei verschiedenen Keimarten gearbeitet, die nahezu überall beim Menschen selbst und in seiner Umwelt zu finden sind:

1. Staphylococus aureus: kleine runde Kugelbakterien mit hoher Resistenz gegenüber trockenem Milieu

2. Candida albicans: dickwandige, grampositive, kapsellose nicht sporenbildende Hefen von ovaler bis rundlicher Form

3. Pseudomonas aeruginosa: Stäbchenbakterien mit hoher Resistenz im feuchten Milieu

Die Begasung wurde wie folgt vorgenommen:
Beaufschlagung der Dosen nur mit 10 bar Preßluft
Beaufschlagung der Dosen nur mit 12 bar $N_2$
Beaufschlagung der Dosen nur mit 12 bar $N_2O$
Kombination Butan + $N_2$
Kombination Butan + $N_2O$
Kombination DME + $N_2$
Kombination DME + $N_2O$

Alle Ergebnisse dieser Versuche waren unbefriedigend, bzw. zeigten, daß eine Keimreduktion mit diesen Mitteln nicht erfolgreich ist.

Die Versuchergebnisse mit den wirksamen Gasen und Gasgemischen sind in Tabelle 1 zusammengefaßt.

Das Mischgas bestand aus einer Mischung von Butan und Dichlordifluormethan im Verhältnis 1 : 3

Die Zahlenwerte sind Logarithmen-Werte, d. h. 5.0 bedeutet $10^5$ Keime pro Gramm Creme, usw.

Die Kontamination fand am 14. November 1988 mit $10^5$ Testkeimen pro Gramm Creme bzw. $10^7$ Testkeimen pro Milliliter Bouillon statt. Die Untersuchungen auf Mikroorganismen fanden am 19.11.1988, am 28.11.1988, am 27.12.1988 und am 27.07.1989 statt.

DME = Dimethylether

Tabelle 1

C R E M E

| Untersuch. /Keimart | ohne Gas | BUTAN allein | +CO$_2$ | MISCHGAS allein | +CO$_2$ | DME allein | +CO$_2$ |
|---|---|---|---|---|---|---|---|
| 19.11.88 | | | | | | | |
| Staph. aureus | 5.0 | <2 | <2 | <2 | <2 | <1 | <1 |
| Pseud. aerug. | 5.0 | <2 | <2 | <2 | <2 | <1 | <1 |
| Cand. albic. | 4.0 | <2 | <2 | <2 | <2 | <1 | <1 |
| 28.11.88 | | Werte unverändert | | | | | |
| 27.12.88 | | Werte unverändert | | | | | |
| 25.07.89 | | | | | | | |
| Staph. aureus | >5.0 | <2 | <2 | <2 | <2 | <1 | <1 |
| Pseud. aerug. | >6.0 | <2 | 4 | 6 | 3.0 | <1 | <1 |
| Cand. albic. | >6.0 | <2 | <2 | <2 | <2 | <1 | <1 |

Tabelle 2

BOUILLON

| Untersuch.-Datum /Keimart | ohne Gas | BUTAN allein | +CO$_2$ | MISCHGAS allein | +CO$_2$ | DME allein | +CO$_2$ |
|---|---|---|---|---|---|---|---|
| 19.11.88 | | | | | | | |
| Staph. aureus | 7.0 ) | für alle war der qualitative Test | | | | | |
| Pseud. aerug. | 7.0 ) | positiv in 1ml Bouillon | | | | | |
| Cand. albic. | 6.0 ) | | | | | | |
| 28.11.88 | | | | | | | |
| Staph. aureus | >7.0 ) | | | | | | |
| Pseud. aerug. | >7.0 ) | 5.0 | 5.0 | 5.0 | 5.0 | 1.0 | 1.0 |
| Cand. albic. | >6.0 ) | | | | | | |
| 27.12.88 | | | | | | | |
| Staph. aureus | >8.0 ) | | | | | | |
| Pseud. aerug. | >8.0 ) | >6.0 | 4.0 | 5.0 | 3.0 | <1.0 | <1.0 |
| Cand. albic. | >7.0 ) | | | | | | |
| 25.07.89 | | | | | | | |
| Staph. aureus | >7.0 | 5.0 | <2.0 | <2.0 | <2.0 | <1.0 | <1.0 |
| Pseud. aerug. | >7.0 | 5.0 | <2.0 | 5.0 | 7.0 | <1.0 | <1.0 |
| Cand. albic. | >7.0 | 7.0 | <2.0 | 6.0 | <2.0 | 3.0 | <1.0 |

Die Tabellen zeigen, daß mit bestimmten Gas-Kombinationen eine hervorragende nicht nur bakteriostatische, sondern auch keimtötende Wirkung erzielt wird. Dies kommt insbesondere mit dem Befund der Tabelle 2 "Bouillon" zum Ausdruck, wo als besonders wirksam die Kombination DME + $CO_2$ dargestellt werden konnte.

In den Kontroll-Dosen ohne Begasung war trotz hoher Ausgangskeimzahl im Verlauf der Versuchsdauer von acht Monaten ein starkes Keimwachstum festzustellen. Dies beweist, daß die Ausgangscreme selbst keine keimtötende Wirkung hatte.

Die dargestellten Ergebnisse wurden durch eine weitere Versuchsreihe bestätigt, bei der bei Cremes und Bouillon eine Kontamination mit abgestuften Keim-Konzentrationen - $19^7$ bis $10^{13}$ Keime pro Gramm Creme bzw. Milliliter Bouillon - durchgeführt wurde.

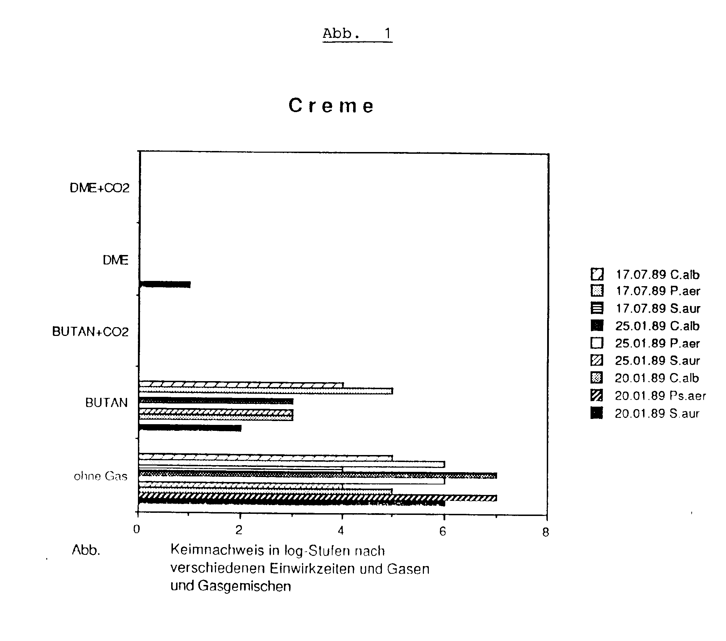

Die Kontamination fand am 16. Januar 1989 statt, die Untersuchungen auf Mikroorganismen am 20.01.1989, 25.01.1989 und 17.07.1989. Die Tabelle 3 und die dazugehörige Abbildung 1 geben Aufschluß über die Ergebnisse.

Tabelle 3

CREME

| Untersuch.-Datum /Keimart | ohne Gas | BUTAN allein | + $CO_2$ | DME allein | + $CO_2$ |
|---|---|---|---|---|---|
| **20.01.89** | | | | | |
| Staph. aureus | 6.0 | 2.0 | nn. | 1.0 | nn. |
| Pseud. aerug. | 7.0 | nn. | nn. | nn. | nn. |
| Cand. albic. | 5.0 | 3.0 | nn. | nn. | nn. |
| **25.01.89** | | | | | |
| Staph. aureus | 4.0 | 3.0 | nn. | nn. | nn. |
| Pseud. aerug. | 6.0 | nn. | nn. | nn. | nn. |
| Cand. albic. | 7.0 | 3.0 | nn. | nn. | nn. |
| **17.07.89** | | | | | |
| Staph. aureus | 4.0 | nn. | nn. | nn. | nn. |
| Pseud. aerug. | 6.0 | 5.0 | nn. | nn. | nn. |
| Cand. albic. | 5.0 | 4.0 | nn. | nn. | nn. |

nn. = nicht nachweisbar

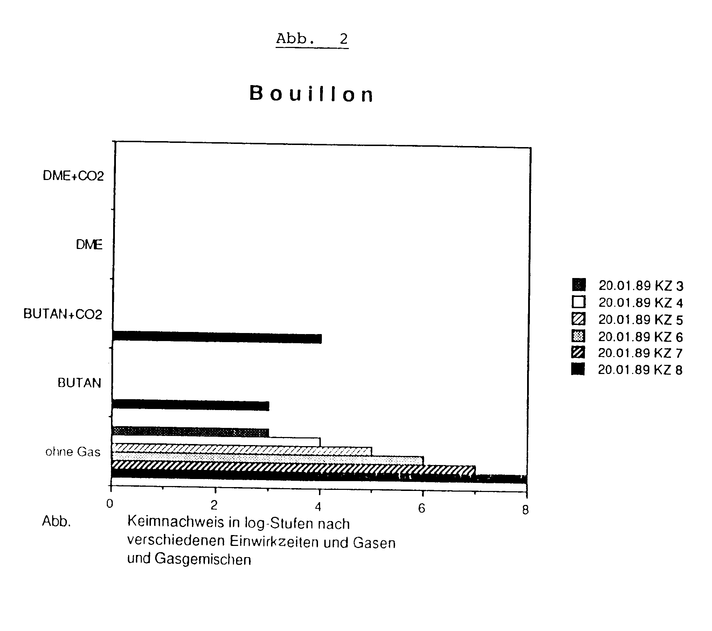

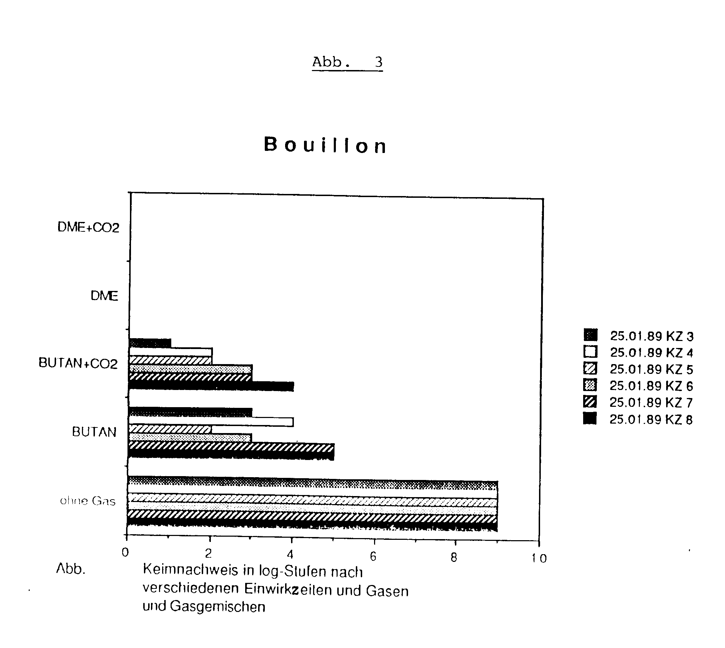

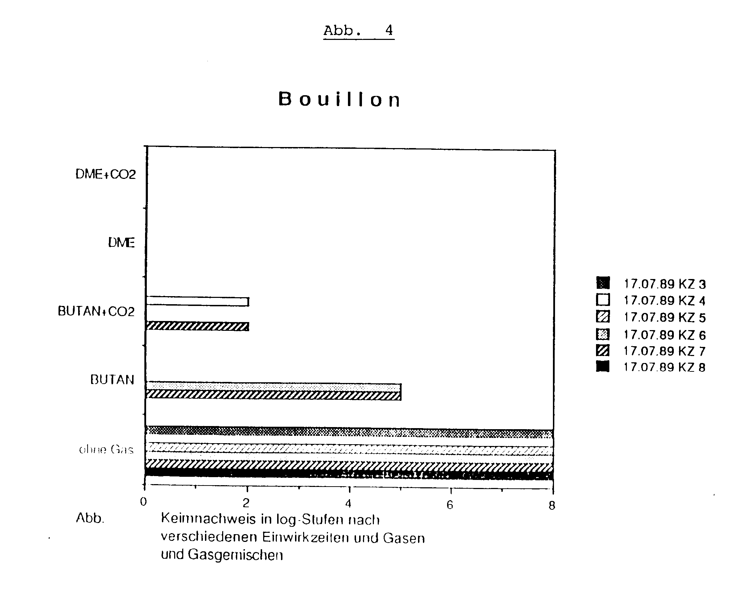

Auch hier erlauben die Ergebnisse mit Bouillon eine differenziertere Aussage. Unter Berücksichtigung der Ausgangskeimzahlen ($10^3$ bis $10^8$) machen die Tabelle 4 "Bouillon" und die dazugehörigen Abbildungen 2, 3 und 4 deutlich, daß Dimethylether (DME) allein und als Gemisch mit $CO_2$ im Gegensatz zu den anderen Gasen und Gasgemischen bereits 24 Stunden nach Begasung und anhaltend über 6 Monate hinweg auch bei einer Ausgangskeim-Konzentration von $10^8$ Keimen pro ml eine vollständige Keimabtötung bewirken.

Tabelle 4

BOUILLON

| Untersuch.-Dat. /Keimart | Ausgangs- Keimzahl | o. Gas | B U T A N | | D M E | |
|---|---|---|---|---|---|---|
| | | | allein | + CO2 | allein | + CO2 |
| 20.01.89 alle 3 Keime (Abb. 2) | 8.0 | 8.0 | 3.0 | 4.0 | nn. | nn. |
| | 7.0 | 7.0 ) | | | | |
| | 6.0 | 6.0 ) | unter der Nachweisgrenze | | | |
| | 5.0 | 5.0 ) | | | | |
| | 4.0 | 4.0 ) | | | | |
| | 3.0 | 3.0 ) | | | | |
| 25.01.89 alle 3 Keime (Abb. 3) | 8.0 | 9.0 | 5.0 | 4.0 | nn. | nn. |
| | 7.0 | 9.0 | 5.0 | 3.0 | nn. | nn. |
| | 6.0 | 9.0 | 3.0 | 3.0 | nn. | nn. |
| | 5.0 | 9.0 | 2.0 | 2.0 | nn. | nn. |
| | 4.0 | 9.0 | 4.0 | 2.0 | nn. | nn. |
| | 3.0 | 9.0 | 3.0 | 1.0 | nn. | nn. |
| 17.07.89 alle 3 Keime (Abb. 4) | 8.0 | 8.0 | nn. | nn. | nn. | nn. |
| | 7.0 | 8.0 | 5.0 | 2.0 | nn. | nn. |
| | 6.0 | 8.0 | 5.0 | nn. | nn. | nn. |
| | 5.0 | 8.0 | nn. | nn. | nn. | nn. |
| | 4.0 | 8.0 | nn. | 2.0 | nn. | nn. |
| | 3.0 | 8.0 | nn. | nn. | nn. | nn. |

nn.= nicht nachweisbar

Die Abbildungen 2, 3 und 4 verdeutlichen die Ergebnisse besonders anschaulich.

Um die Wirkung des Systems weiter zu überprüfen, wurde das Keimspektrum erweitert und einige besonders resistente Keimarten mit eingebracht.

Außer den drei obengenannten Keimen wurden noch die folgenden Arten eingesetzt:

Aspergillus niger: schwarzer Kolben-Schimmel Mycobacterium terrae: zur Gruppe der Tuberkulose-Bakterien zugehörig mit hoher Umweltresistenz.

Bacillus subtilis: weit verbreitete Stäbchen mit schnellem Wachstum mit der Fähigkeit zur Sporenbildung, d. h. resistente Überlebensform.

Bacillus sterothermophilus: auch wegen der Fähigkeit zur Sporenbildung extrem widerstandsfähig, dient als Testkeim für Autoklaven.

Selbst für diese teilweise sehr resistenten Keimarten konnte mit Dimethylether bzw. Dimethylether + $CO_2$ zum Teil eine vollständige Abtötung zumindest aber eine Reduktion um 5 bis 6 log-Stufen erreicht werden.

Deutlich geringere Wirkungen waren mit Butan bzw. Butan + $CO_2$ zu erreichen.

Die Ergebnisse zeigen deutlich, daß mit den obengenannten Verfahren die Herstellung und Erhaltung der Keimfreiheit bei kosmetischen und medizinischen Produkten ohne Zusatz chemischer Konservierungsmittel möglich ist.

In weiteren Untersuchungen wurde der Einsatz dieses Verfahrens zur Keimfreiheitmachung, d.h. zur Sterilisation von Festkörpern, insbesondere von medizinischen Instrumenten und Geräten überprüft.

Versuchsanordnung:

In einen - mit $CO_2$ - entlüfteten Druckbehälter wurden mit den weiter unten angegebenen Keimen kontaminierte Pinzetten und Kunststoffplättchen gegeben und die Behälter mit 3,5 Vol.-% bzw. 75, Vol-% einer 0,9%-igen Kochsalzlösung aufgefüllt. Danach wurde der Behälter druckfest verschlossen und etwa 70% des Behältervolumens wurde mit den jeweiligen Flüssiggasen gefüllt.

Bei den Flüssiggasen wurden wieder folgende Kombinationen überprüft:

Butan allein bzw. mit $CO_2$ und

DME allein bzw. mit $CO_2$.

Die Pinzetten und Kunststoffplättchen wurden mit den nachstehenden Keimlösungen kontaminiert.

| Keimart: | Ausgangskeimzahl: |
|---|---|
| Staphylococcus aureus | $1,0 \times 10^7$ |
| Candida albicans | $7,1 \times 10^6$ |
| Pseudomonas aeruginosa | $1,1 \times 10^8$ |
| Aspergillus niger | $3,0 \times 10^6$ |
| Bacillus subtilis | $1,3 \times 10^7$ |
| E. Coli | $2,0 \times 10^8$ |

Ergebnisse:

Bei allen untersuchten Gaskombinationen konnte für alle Keime eine Reduktion von mindestens 4 log-Stufen festgestellt werden.

Bei den Kombinationen DME + $CO_2$ + 3,5 V-% NaCl 0,9%-ig sowie DME + 7,5 V-% NaCl 0,9%-ig konnten bereits nach 23 Stunden Einwirkzeit keinerlei Keime mehr nachgewiesen werden.

Diese Ergebnisse zeigen deutlich, daß die für Cremes und Lösungen nachgewiesene Abtötung von Mikroorganismen und resistenten Keimprodukten auch durch das obengenannte Verfahren bei kontaminierten Festkörpern erzielt werden kann.

Die angewandten Drucke bewegten sich zwischen 3 und 20 bar.

**Patentansprüche**

1. Verfahren zum Abtöten von Keimen, Keimprodukten, infektiösen Partikeln und dergleichen pathogenen Erregern auf Festkörpern sowie in Zubereitungen verschiedener Zusammensetzungen und zum Haltbarmachen dieser Zubereitungen, insbesondere von kosmetischen, medizinischen und pharmazeutischen Erzeugnissen, wobei man die Festkörper oder Zubereitungen in einen Druckgasbehälter mit Ventil einbringt, bzw. einfüllt,
**dadurch gekennzeichnet**, daß der Behälterinhalt mit Butan oder Dimethylether, oder Butan und Dimethylether, oder Butan und Kohlendioxid, oder Dimethylether und Kohlendioxid, oder Butan, Dimethylether und Kohlendioxid begast, wird.

2. Verfahren nach Anspruch 1, dadurch gekennzeichnet, daß man bei Begasen von Zubereitungen FLüssiggase in einer Menge von 5 bis 20 Gew.-%, bezogen auf das Gewicht der Zubereitung, zugibt.

3. Verfahren nach Anspruch 1 und 2, dadurch gekennzeichnet, daß man die Zubereitung mit einem Druck von 3 bis 20 bar beaufschlagt.

4. Verfahren nach Anspruch 1, daß man den oder die Festkörper im Druckgasbehälter in Gegenwart einer Flüssigkeit mit Flüssiggas umspült und begast.

5. Verfahren nach Anspruch 1 und 4, dadurch gekennzeichnet, daß man mit dem Gas oder Gasgemisch mit einem Druck von 3 bis 20 bar beaufschlagt.

**Claims**

1. A process for killing cells, cellular products, infectious particles and similar pathogens on solid objects as well as in preparations of various components and for conserving these preparations, in particular for cosmetic, medicinal and pharmaceutical products, whereby the solid objects are placed and the preparations are filled into a pressure container equipped with a valve,
**characterized in that** said pressure container is attacked with a gas atmosphere cosisting of butane or dimethyl ether, or butane and dimethyl ether, or butane and carbon dioxide, or dimethyl ether and carbon dioxide, or butane, dimethyl ether and carbon dioxide.

2. A process according to claim 1, characterized in that the preparations are attacked with gas by adding liquid gases in an amount of 5 to 20% by weight with respect to the weight of the preparation.

3. A process according to claim 1 and 2, characterized in that the preparation is attacked with a gas pressure of 3 to 20 bar.

4. A process according to claim 1, charaterized in that the solid object- or objects - in the pressure container is - are - rinsed and attacked with liquid gas in the presence of a liquid.

5. A process according to claim 1, characterized in that the gas or gas mixture is added with a pressure of 3 to 20 bar.

## Revendications

1. Procédé pour tuer des germes, des produits donnant des germes, des particules infectieuses et des agents pathogènes analogues sur des solides ainsi que dans des préparations de compositions diverses et pour stériliser ces préparations, notamment de produits cosmétiques, médicaux et pharmaceutiques, qui consiste à placer ou à verser les solides ou les préparations dans un récipient résistant à du gaz sous pression et muni d'une vanne,
**caractérisé en ce qu'**il consiste à mettre le contenu du récipient sous une atmosphère gazeuse constituée de butane ou d'oxyde diméthylique, ou de butane et de dioxyde de carbone, ou d'oxyde diméthylique et de dioxyde de carbone, ou de butane, d'oxyde diméthylique et de dioxyde de carbone.

2. Procédé selon la revendication 1, caractérisé en ce qu'il consiste à ajouter, lors de la mise des préparations sous atmosphère gazeuse, des gaz liquéfiés en une quantité représentant de 5 à 20 % du poids de la préparation.

3. Procédé suivant la revendication 1 ou 2, caractérisé en ce qu'il consiste à injecter la préparation sous une pression de 3 à 20 bar.

4. Procédé suivant la revendication 1, caractérisé en ce qu'il consiste à balayer le ou les solides dans le récipient résistant à du gaz sous pression et à les mettre sous une atmosphère gazeuse à l'aide d'un gaz liquéfié en présence d'un liquide.

5. Procédé suivant la revendication 1 ou 4, caractérisé en ce qu'il consiste à injecter le gaz ou le mélange gazeux sous une pression de 3 à 20 bar.

Abb.   1

# C r e m e

Legend:
- 17.07.89 C.alb
- 17.07.89 P.aer
- 17.07.89 S.aur
- 25.01.89 C.alb
- 25.01.89 P.aer
- 25.01.89 S.aur
- 20.01.89 C.alb
- 20.01.89 Ps.aer
- 20.01.89 S.aur

Abb.   Keimnachweis in log-Stufen nach verschiedenen Einwirkzeiten und Gasen und Gasgemischen

Abb.   2

# B o u i l l o n

20.01.89 KZ 3
20.01.89 KZ 4
20.01.89 KZ 5
20.01.89 KZ 6
20.01.89 KZ 7
20.01.89 KZ 8

Abb.   Keimnachweis in log-Stufen nach verschiedenen Einwirkzeiten und Gasen und Gasgemischen

Abb.    3

# B o u i l l o n

DME+CO2

DME

BUTAN+CO2

BUTAN

ohne Gas

0    2    4    6    8    1 0

Abb.    Keimnachweis in log-Stufen nach
verschiedenen Einwirkzeiten und Gasen
und Gasgemischen

■ 25.01.89 KZ 3
□ 25.01.89 KZ 4
▨ 25.01.89 KZ 5
▨ 25.01.89 KZ 6
▨ 25.01.89 KZ 7
■ 25.01.89 KZ 8

Abb. 4

# Bouillon

Abb. Keimnachweis in log-Stufen nach
verschiedenen Einwirkzeiten und Gasen
und Gasgemischen